# EUROPEAN PATENT SPECIFICATION

(11) **EP 4 302 841 B1**
(45) Date of publication and mention of the grant of the patent: **12.08.2026**
(21) Application number: 23181137.3
(22) Date of filing: 23.06.2023
(51) Int. Cl.: A63B 22/02, A63B 24/00, A63B 71/06

(54) **METHOD FOR DETECTING THE DISTANCE OF PORTIONS OF THE BODY OF A USER FROM A PART OF A GYMNASTIC MACHINE AND GYMNASTIC MACHINE CARRYING OUT THE METHOD**
VERFAHREN ZUR ERFASSUNG DES ABSTANDS VON KÖRPERTEILEN EINES BENUTZERS VON EINEM TEIL EINES GYMNASTISCHEN GERÄTES UND DAS VERFAHREN DURCHFÜHRENDES GYMNASTISCHES GERÄT
PROCÉDÉ DE DÉTECTION DE LA DISTANCE DE PARTIES DU CORPS D'UN UTILISATEUR D'UNE PARTIE D'UNE MACHINE DE GYMNASTIQUE ET MACHINE DE GYMNASTIQUE METTANT EN OEUVRE LE PROCÉDÉ

(30) Priority: 05.07.2022 IT 202200014224
(43) Date of publication of application: 10.01.2024
(73) Proprietor: Technogym S.p.A., 47521 Cesena (FC) (IT)
(72) Inventor: VANNONI, Enrico, 47521 Cesena (FC) (IT)
(74) Representative: Tiburzi, Andrea

(56) References cited:
- EP-A2- 0 941 746
- US-A- 5 314 391
- US-A- 6 135 924
- US-A1- 2019 240 535
- US-A1- 2022 111 247
- US-B2- 10 016 656

## Description

The present invention relates to a method of detecting the distance of portion of the body of a user from a part of a gymnastic machine, in particular of a treadmill.

The present invention also relates to a computer program comprising instructions which, when the program is executed by a computer, cause the computer to carry out the steps of the method; to a computer-readable storage medium comprising the computer program; and to a gymnastic machine which implements the method.

### Field of invention

More in detail, the invention relates to a method of the aforementioned type, designed and implemented in particular for detecting the distance of several portions of the body of a user, with respect to an upper supporting structure of the treadmill, but which can be used for any gymnastic machine, in which it is necessary to detect the distance of portions of the body of a user.

In the following, the description will be directed to a method implemented in a treadmill machine, during the execution of a walk or a run by a user, but it is clear that the same should not be considered limited to this specific use.

### Prior art

As is well known, gymnastic machines with rotating belts are currently known, comprising an exercise surface, which can be a belt or flat carpet, or which can comprise a plurality of strips so as to form a flat or curved "rolling shutter", in which measurements of a user's running progress are made in order to determine the quality of the training.

It is in fact of great importance to be able to determine the quality of the training of the user, so that it is on one hand performing, and on the other hand also reliable and safe, for the well-being and health of the user.

Methods for determining the training quality, which requires very complicated and sophisticated detection devices that can cause errors in data collection and processing, are currently known.

The prior art also comprises the documents US 2022/111247 A1, US 6135924 A, US 10016656 B2, EP 0941746 A2, US 5314391 A, and US 2019/240535 A.

### Scope of the invention

In light of the above, it is, therefore, a scope of the present invention to provide a method that allows to at least partially overcome the drawbacks reported with reference to the prior art, and which therefore allows reading values relating to several portions of the body of the user by means of structurally simple devices, which allow the quality of the training to be determined in a precise and reliable way.

A further scope of the present invention is to provide a gymnastic machine required for carrying out the method and the apparatuses that carries out this method.

### Object of the invention

It is, therefore, specific object of the present invention a method for detecting the distance of portions of the body of a user from a front part of a gymnastic machine provided with a detecting device, arranged on said front part and a logic control unit, operatively connected to said detecting device, during the execution of a gymnastic exercise on an exercise surface, said method comprising the following steps:
- detecting, by means of said detecting device, a plurality of distance values of at least one portion of the body of said user from said front part of said gymnastic machine;
- processing, by means of said logic control unit, at least one first group of said plurality of distance values of at least one portion of the body of said user, for calculating the distance of at least one portion of the chest of the user from said detecting device;
- and processing, by means of said logic control unit, at least one second group of said plurality of distance values of at least one portion of the body of said user, for calculating the distance of at least one portion of the right leg of the user from said detecting device;
- and processing, by means of said logic control unit, at least one third group of said plurality of distance values of at least one portion of the body of said user, for calculating the distance of at least one portion of the left leg of the user from said detecting device.

Further according to the invention, said processing step comprises the following sub-steps:
- selecting, by means of said logic control unit, said first group of said plurality of distance values of at least one portion of the body;
- calculating, by means of said logic control unit, said distance of at least one portion of the chest of the user from said detecting device as a statistical data of said first group of values of said plurality of distance values of at least one portion of the body.

Still according to the invention, said method comprises the following step:
- comparing, by means of said logic control unit, said current distance data of at least one portion of the chest, calculated in said sub-step of calculating, with a predetermined reference value, corresponding to an optimal distance of the user from said detecting device, on said exercise surface.

Preferably according to the invention, said step of comparing comprises the following sub-steps:
- if said current distance data of at least one portion of the chest is higher than said predetermined reference value plus a predetermined threshold value, then commanding the decrease, by means of said logic control unit, of the advancement speed of said exercise surface,
- if said current distance data of at least one portion of the chest is included in a range having as lower bound said predetermined reference value minus said predetermined threshold value and as upper bound said predetermined reference value plus said predetermined threshold value, then leave unchanged the advancement speed of said exercise surface;
- if said current distance data of at least one portion of the chest is lower than said predetermined reference value minus a predetermined threshold value, then command the increase, by means of said logic control unit, of the advancement speed of said exercise surface.

Always according to the invention, said processing step comprises the following sub-steps:
- selecting, by means of said logic control unit, said second group of values of said plurality of distance values of at least one portion of the body;
- calculating, by means of said logic control unit, said distance of at least one portion of the right leg of the user from said detecting device as a statistical data of said second group of values of said plurality of distance values of at least one portion of the body.

Further according to the invention, said processing step comprises the following sub-steps:
- selecting, by means of said logic control unit, said third group of values of said plurality of distance values of at least one portion of the body;
- calculating, by means of said logic control unit, said distance of at least one portion of the left leg of the user from said detecting device as a statistical data of said third group of values of said plurality of distance values of at least one portion of the body.

Still according to the invention, said method comprises the following step:
- providing a display of the parameters of the execution of the gymnastic exercise, based on the distance data calculated in said sub-steps of calculating.

Preferably according to the invention, said step of providing a display comprises the following sub-steps:
- obtaining the oscillation period of the right leg, from said distance data of at least one portion of the right leg;
- obtaining the oscillation period of the left leg, from said distance data of at least one portion of the right leg;
- comparing said oscillation period of the right leg and said oscillation period of the left leg;
- displaying the result of said comparison by means of a display of said gymnastic machine.

Still according to the invention, said step of providing a display comprises the following sub-steps:
- obtaining the amplitude of the oscillation of the left leg, from said distance data of at least one portion of the left leg;
- obtaining the amplitude of the oscillation of the right leg, from said distance data of at least one portion of the right leg;
- comparing said amplitude of the oscillation of the right leg and said amplitude of the oscillation of the left leg;
- displaying said amplitude of the oscillation of the right leg and said amplitude of the oscillation of the left leg or the result of said comparison by means of a display of said gymnastic machine.

Further according to the invention, said step of providing a display comprises the following sub-steps:
- obtaining the oscillation period of the right leg, from said distance data of at least one portion of the right leg;
- obtaining the oscillation period of the left leg, from said distance data of at least one portion of the left leg;
- calculating the running cadence as the inverse of the time that passes between two successive peaks of the oscillation of the right leg and of the oscillation of the left leg;
- displaying said cadence by means of a display of said gymnastic machine.

Still according to the invention, said method comprises the following steps:
- detecting a plurality of ambient brightness values;
- processing said plurality of detected ambient brightness values for calculating an ambient brightness index;
- comparing, said ambient brightness index, with a predetermined threshold value;
- if said ambient brightness index is greater than said predetermined threshold value, carrying out the steps of the method;
- if said ambient brightness index is lower than said predetermined threshold value, activating a safety operating state for said gymnastic machine.

Preferably according to the invention, in said step of detecting, said plurality of distance values of at least one portion of the body of said user is detected in the form of a matrix, in which at least one distance value is associated with each cell.

Still according to the invention, said first group of said plurality of distance values of at least one portion of the body of said user is contained in an upper portion of said matrix.

Further according to the invention, said second group of said plurality of distance values of at least one portion of the body of said user is contained in a first lower portion of said matrix.

Still according to the invention, said third group of said plurality of distance values of at least one portion of the body of said user is contained in the cells of a second lower portion of said matrix.

A further object of the present invention is a computer program comprising instructions that, when said program is executed by a computer, cause the execution by said processor of the steps of said method.

A further object of the present invention is a computer-readable storage medium, comprising the computer program.

A further object of the present invention is a gymnastic machine comprising an exercise surface for the training of a user on said gymnastic machine, the exercise surface having a development direction and an advancement direction parallel to the development direction of the exercise surface, a logic control unit of the movement of the exercise surface, a detecting device, operatively connected to said logic control unit, configured to detect at least one distance value of a portion of the user from said detecting device itself, during the execution of the gymnastic exercise by the user on the exercise surface, said logic control unit being configured to carry out the method for detecting the distance of portions of the body of a user from a part of a gymnastic machine, according to the method.

### Brief description of the figures

The present invention will be now described, for illustrative but not limitative purposes, according to its preferred embodiments, with particular reference to the figures of the enclosed drawings, wherein:
figure 1 shows a side view of a first embodiment of the gymnastic machine that implements the method, object of the present invention;
figure 2 shows a top view of the gymnastic machine of figure 1;
figure 3 shows a perspective view of a second embodiment of the gymnastic machine that implements the method, object of the present invention;
figure 4 shows a top view of the gymnastic machine in figure 3;
figure 5 shows a detecting matrix of a component of the gymnastic machine;
figure 6 shows a block diagram of the method of detecting the distance of portions of the body of a user from a part of a gymnastic machine, object of the present invention; and
figure 7 shows the trend of signals detected in the gymnastic machine during the execution of the method.

### Detailed description

In the various figures, similar parts will be indicated with the same reference numbers.

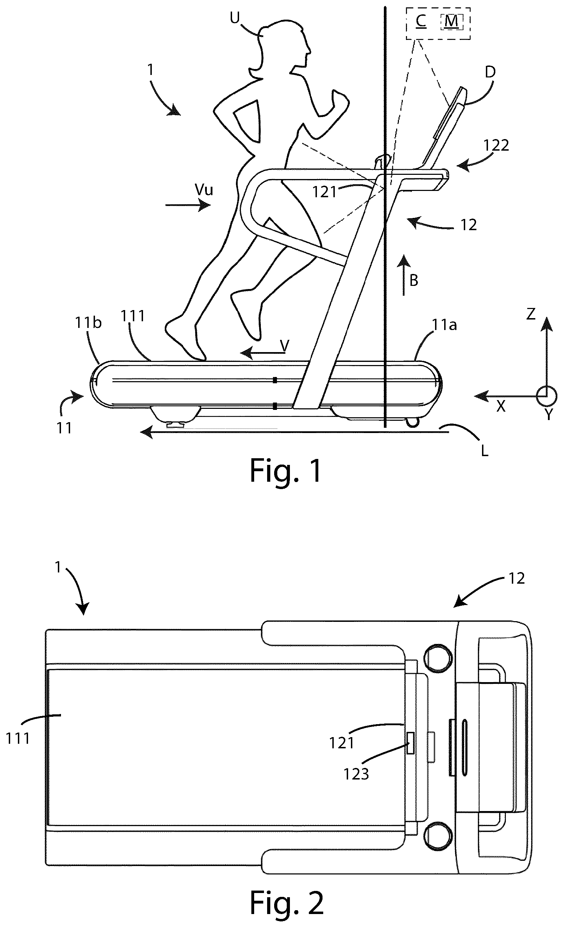

With reference to figure 1, the gymnastic machine 1, which implements the method object of the present invention, is in particular a treadmill.

Said treadmill 1 has a supporting base 11 which extends along a longitudinal axis L, parallel to a first Cartesian axis X of a Cartesian reference system XYZ shown in the figure.

Said supporting base 11 has a first end 11ₐ and a second end 11_{b}.

In the first end 11ₐ a first rotating element is arranged, not shown in the figure, capable of rotating about a first axis of rotation parallel to a second Cartesian axis Y of the Cartesian reference system.

In the second end 11_{b} a second rotating element is arranged, not shown in the figure, capable of rotating about a second axis of rotation parallel to said second Cartesian axis Y of the Cartesian reference system.

Said treadmill 1 comprises an exercise surface 111 for the training of the user U, such as running or walking, or any other exercise that can be performed on said exercise surface 111.

In a first embodiment, said exercise surface 111 is a carpet or a belt.

Said exercise surface 111 is operatively connected to said first and second rotating element, so that, when said first and second rotating elements rotate about the respective first and second axes, said exercise surface 111 is capable of sliding according to an advancement direction V, parallel to said first Cartesian axis X.

Said advancement direction V is opposite to the moving direction Vᵤ of the user U, during the execution of the gymnastic exercise.

In said embodiment, said treadmill comprises a motor, not shown in the figure, capable of rotating said first or said second rotating element, or both, so as to cause the sliding of said exercise surface 111.

Furthermore, said treadmill 1 comprises a logic control unit C, arranged on said treadmill 1, or remote.

Said logic control unit C is capable of controlling the movement of said exercise surface 111.

In particular, said logic control unit C is capable of controlling the operation of said motor.

Furthermore, said logic control unit C comprises a memory unit M, in which parameters and operating data for the operation of the treadmill 1 are stored.

Said treadmill 1 comprises a supporting frame 12, arranged at said first end 11ₐ of said supporting base 11.

Said supporting frame 12 extends according to an axis B substantially orthogonal to the exercise surface 111 of said supporting base 11, and parallel to a third Cartesian axis Z of the Cartesian reference system.

Said supporting frame 12 comprises one or more uprights and tubular elements connected to each other, on which the user U can **rest** his upper limbs during training or in the rest step.

In particular, said supporting frame 12 comprises a front part 121 that faces the user U, which can be equipped with housings for supporting objects of various types.

Said supporting frame 12 further comprises a user interface 122, operatively connected to said logic control unit C.

Said user interface 122 is configured to allow the user to issue commands to said treadmill 1.

Said user interface 122 can comprise a touchscreen display D or a display D connected to a keyboard or other data entry devices for user U, not shown in the figure.

The display D allows the user U to view both specific contents of the use of the treadmill 1, including contents related to the method of detecting the distance of the portions of the body that will be described below, and multimedia contents of entertainment or services for user U.

With reference now to figure 2, said treadmill 1 comprises a detecting device 123 for the distance of portions of the body of the user U.

Said detection device 123 is operatively connected to said logic control unit C.

Said detecting device 123 is arranged on said supporting frame 12.

In particular, in the preferred embodiment that is being described, said detecting device 123 is arranged in said front part 121.

However, without departing from the scope of protection of the present invention, said detecting device 123 can also be arranged in different positions, such as for example in the lateral or rear part of said supporting frame 12 of said treadmill 1.

Said detecting device 123 is a distance sensor, configured to detect a distance value O of a portion of the user U from himself, during the training on the treadmill 1.

Portion of the body of the user U here means at least a portion of the chest, and right leg, and left leg of the user U.

In particular, the operation of said distance sensor 123 is based on the time-of-flight principle, or the measurement of the distance O between the sensor and the user U is based on the difference of time between the emission of a signal and its return to the sensor, after being reflected by the user U.

The signal can be of electromagnetic or acoustic nature, especially light or sound.

In the embodiment described, the sensor used is of the optical type, therefore the signal used is of an electromagnetic nature.

The distance sensor 123 detects values and outputs a packet of values comprising several distance values O and/or any further values.

In particular, in this embodiment, the distance sensor 123 outputs a packet of values in the form of a matrix M.

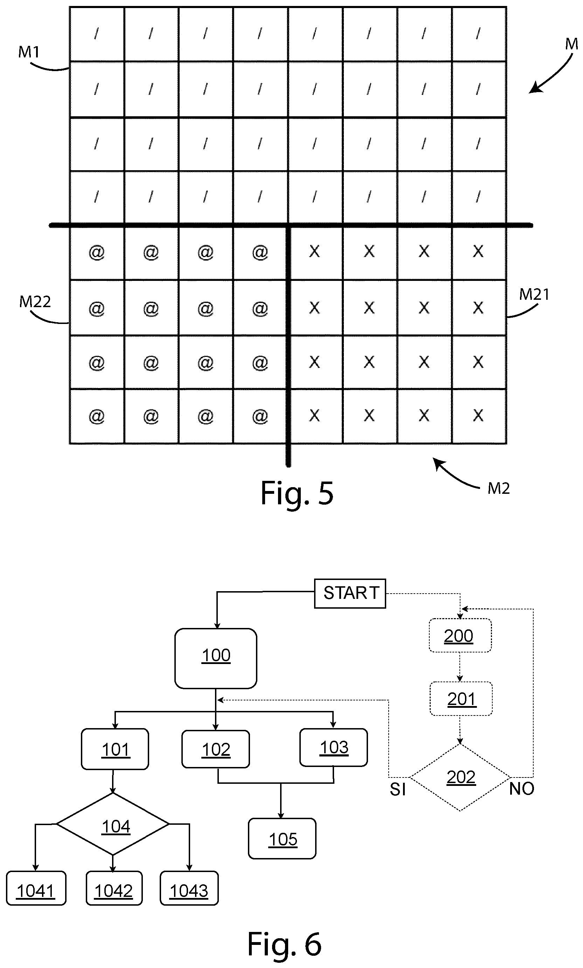

With reference to figure 5, in particular, said matrix M has a size of 8x8 cells and will be described in detail below.

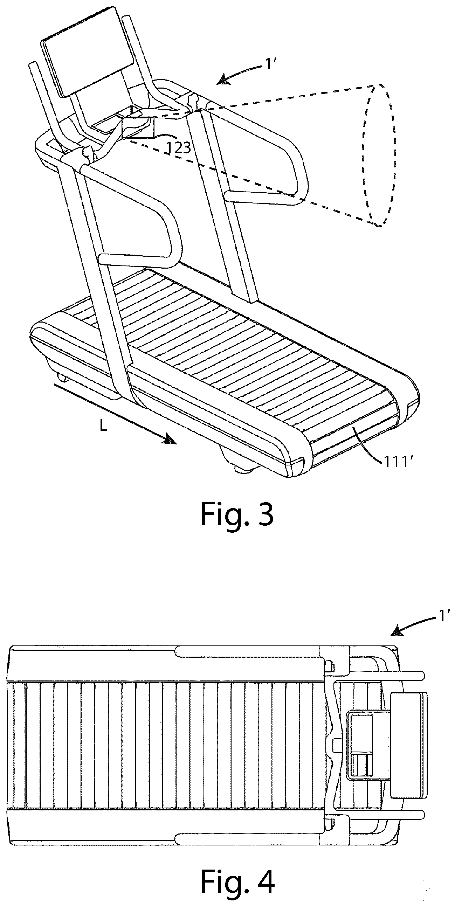

With reference to figures 3 and 4, in a second embodiment, said gymnastic machine 1' varies if compared to the first embodiment described above, only for the exercise surface 111'.

In the second embodiment, said exercise surface 111' comprises a plurality of strips arranged transversely to the longitudinal axis L, giving the exercise surface 111' a so-called "roller shutter" shape.

With reference to figure 6, the method of detecting the distance O of portions of the body of a user U from a part of a gymnastic machine 1, object of the present invention, provides the execution of the following steps, by means of said logic control unit C.

The method implements the calculation of the distance of at least a portion of the chest O₁ of the user U from the distance sensor 123, and the calculation of the distance of a portion of the right leg O₂ and of the left leg O₃ of the user U, from the distance sensor 123.

The method is performed by said logic control unit C, with the values sent by said distance sensor 123 to said logic control unit C.

In particular, as previously described, the portions of the body of the user U are at least a portion of the chest, right leg, and left leg.

Furthermore, by part of the gymnastic machine 1 we mean the supporting frame 12, in particular the front part 121.

The method involves the execution of the following steps.

In a detecting step 100, said distance sensor 123 detects the distance O of said user U in the form of a matrix M.

In particular said matrix M has a dimension of 8x8 pixels.

In particular, each cell of the matrix M is associated with a distance value O measured by a portion of the body of the user U.

Each cell of the matrix M can also be associated with an ambient brightness index I_{L}.

The sampling frequency of the distance sensor 123 can vary from 5 to 20 times per second, and, for each detection, the values are collected and the matrix M is generated.

In a first processing step 101 the values of said matrix M are processed.

In particular, in a first sub-step 1011 the distance of a portion of the chest O₁ of the user U is calculated from the distance sensor 123, by selecting a first group of detected distance values.

In particular, an upper portion M1 of the matrix M, which contains said first group of detected distance values, is selected.

In one embodiment, the dimensions of said upper portion M1 are 4x8 pixels or cells.

Without departing from the scope of protection of the present invention, the dimensions of said upper portion M1 may have different values.

For example, the dimensions of said upper portion M1 can be 5x8 or 6x8.

As shown in figure 5, each cell of the upper portion M1 contains the distance values of a portion of the chest O₁ of the user, indicated with the symbol "/".

In a second sub-step 1012 a statistical datum of the distance of a portion of the chest O₁ measured from each cell of the upper portion M₁ of the matrix M for each sampling cycle, i.e. for each time instant, is calculated.

Said statistical datum can be, for example, the mode, median, or mean, which represents the statistical distribution of the first group of distance values in the upper portion M₁ of the matrix M.

In a comparing step 104, the current distance value of a portion of the chest O₁ is compared with a reference value O_{1R}, which corresponds to an optimal positioning distance of the user U on said exercise surface 111.

If the current distance value of a portion of the chest O₁ is higher than the reference value O_{1R} plus a predetermined threshold value S, i.e., if O₁ > O_{1R} + S, then in a first controlling step 1041, said logic control unit C is configured to determine the decrease of the advancement speed of the exercise surface 111.

If the current distance value of a portion of the chest O₁ is within a range determined by O_{1R} - S < O₁ < O_{1R} + S, then in a second controlling step 1042, said logic control unit C is configured to leave unchanged the advancement speed of the exercise surface 111.

If the current distance value of a portion of the chest O₁ is lower than the reference value O_{1R} minus a predetermined threshold value S, i.e. if O₁ < O_{1R} - S, then in a third controlling step 1043, said logic control unit C is configured to determine the increase of the advancement speed of the exercise surface 111.

The method also involves the execution of the following steps.

Still referring to figures 5 and 6, after said detecting step 100, the values of said matrix M are processed, in a second processing step 102, selecting a second group of detected distance values.

In particular, in a first sub-step 1021, the distance of a portion of the right leg O₂ of the user U from distance sensor 123 is calculated.

In order to determine a value of the distance of a portion of the right leg O₂, a first lower portion M₂₁ of said matrix M, which contains said second group of detected distance values, is selected.

In particular, the dimensions of said first lower portion M₂₁ are 4x4 pixels.

Without departing from the scope of protection of the present invention, the dimensions of said first lower portion M₂₁ may have different values.

For example, the dimensions of said first lower portion M₂₁ can be 4x5 or 4X3.

As shown in figure 5, each cell of the first lower portion M21 contains the distance values of a portion of the right leg O₂ of the user, indicated with the symbol "X".

In a second sub-step 1022, it is calculated a statistical datum of the distance of a portion of the right leg O₂ measured from each cell of the first lower portion M₂₁ of the matrix M for each sampling cycle, i.e., for each time instant.

Said statistical datum can be, for example, the mode, median or mean, which represent the statistical distribution of the first group of distance values in the upper portion M₁ of the matrix M.

Still referring to figures 5 and 6, after said detecting step 100, in a third processing step 103, the values of said matrix M are processed, selecting a third group of detected distance values.

In particular, in a first sub-step 1031, it is calculated the distance of a portion of the left leg O₃ of the user U from the distance sensor 123.

A second lower portion M22 of said matrix M is selected, in order to determine a value of the distance of a portion of the left leg O₃.

In particular, the dimensions of said second lower portion M22 are 4x4 pixels.

Without departing from the scope of protection of the present invention, the dimensions of said second lower portion M22 may have different values.

For example, the dimensions of said second lower portion M22 can be 4x5 or 4X3.

As shown in figure 5, each cell of the second lower portion M22 contains the third group of distance values of a portion of the left leg O₃ of the user, indicated with the symbol "@".

In a second sub-step 1032, a statistical datum of the distance of a portion of the left leg O₃, measured by each cell of the second lower portion M22 of the matrix M for each sampling cycle, is calculated, i.e. for each time instant.

Said statistical datum can be, for example, the mode, median or mean, which represent the statistical distribution of the third group of distance values in the second lower portion M22.

In an evaluating step 105, said distances of a portion of the right leg O₂ and a porti on of the left leg O are processed, in order to provide the user information on the characteristics and quality of the execution of the exercise in real-time, by means of said display D.

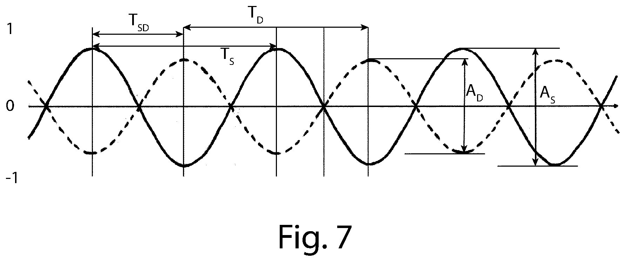

Alternatively, with reference to figure 7, memorizing, in said memory unit M of said logic control unit **C,** the trend of the distance values of a portion of the right leg O₂, indicated in the figure with a dashed line trace, and the distance values of a portion of the left leg O₃, indicated in the figure with a continuous line, cyclic and out-of-phase curves are obtained, from which it is possible to obtain characteristic parameters.

In particular, the characteristic parameters are: the oscillation amplitude of the right leg, indicated with A_{D}, the oscillation amplitude of the left leg, indicated with A_{S}, the oscillation period of the right leg, indicated with T_{D} and the oscillation period of the left leg, indicated with T_{S}.

By calculating the distance between two succeeding peaks T_{SD} of the right leg oscillation period T_{D} and the left leg oscillation period Ts, the value of the cadence C=1/T_{SD}, which indicates the number of steps taken in one minute, is obtained.

The cadence C can be compared with recommended reference values based on the height or other anthropometric parameters of the user U, in order to provide the user with suggestions on how to improve the quality of the run, by means of this display D.

Comparing the periods T_{D} e Ts and the oscillations A_{D} e As with pre-established experimental data, indications for the user U on the quality of training can be displayed, such as the symmetry of use of the lower limbs during running or the detection of asymmetry of these.

Examples of visualizations can be arrows or indicators that indicate the deviation of the calculated values from a symmetry condition.

For example, the greater the oscillation period T_{D} compared to the oscillation period T_{S}, or vice versa, the less symmetrical the run of the user will be.

In an optional step 200, a brightness control step of the values detected by the distance sensor 123 can also be performed.

A valuation of the ambient brightness index I_{L} is carried out, which is the number of photons received by the pixels of the distance sensor 123.

As previously described, the cells of said matrix M, in addition to detecting distance values, also memorize ambient brightness values I_{L.}

In a processing step 201, said ambient brightness values I_{L} detected by said matrix M are processed.

In a verification step 202, the ambient brightness values I_{L} processed in said processing step 201 are compared with a predetermined threshold value.

Said pre-established threshold value relates to a determined distance sensor 123 used.

If the ambient brightness index I_{L} is greater than said pre-established threshold value, then some optional automatic functions, which can be performed by said treadmill 1, such as the control of the rotation speed of said exercise surface 111, are enabled.

If the ambient brightness index I_{L} is lower than said pre-established threshold value, said treadmill 1 enters a "safety" operating state, which provides for disabling some accessory automatic functions that can be performed by said treadmill 1 itself.

In particular, if the ambient brightness data I_{L} processed are higher than said pre-established threshold value, then it is proceed with the execution of the steps of the method starting from said detecting step 100.

If the ambient brightness data I_{L} processed are lower than said pre-established threshold value, then the steps of the method starting from said detecting step 100 are disabled.

The operation of the treadmill 1 described above is as follows.

When a user U intends to perform a workout, s/he accesses said treadmill 1 exercise surface 111.

By means of the display D s/he actuates the operation, and the motor rotates said first rotating element about its rotation axis.

The rotation of the first rotating element causes the rotation of said exercise surface 111, which also rotates the second rotating element around its rotation axis.

The user U can then perform the walking or running gymnastic exercise.

Said distance sensor 123 detects said distance values O of portions of the user U and sends said processed values to said logic control unit C, which follows the steps of the method described above.

Embodiments related to the processing of a single body part (e.g. chest or legs alone) are not encompassed by the wording of the claims but are considered as useful for understanding the invention.

### Advantages

As is apparent from the above description, the method object of the present invention allows detecting and determining the distance of different portions of the body of a user from a part of a gymnastic machine, during the performance of a gymnastic exercise.

Furthermore, following the determination of said distances, the method allows actuating some controls on said machine.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. A method for detecting the distance of portions of the body of a user (U) from a front part (121) of a gymnastic machine (1) provided with a detecting device (123), arranged on said front part (121) and a logic control unit (C), operatively connected to said detecting device (123), during the execution of a gymnastic exercise on an exercise surface (111) configured to move along an advancement direction at an advancement speed, said method comprising the following steps:
- detecting (100), by means of said detecting device (123), a plurality of distance values of at least one portion of the body of said user (U) from said front part (121) of said gymnastic machine (1), said plurality of distance values of at least one portion of the body of said user (U) being detected in the form of a matrix (M), in which at least one distance value (O) is associated with each cell;
- processing (101), by means of said logic control unit (C), at least one first group of said plurality of distance values of at least one portion of the body of said user (U), for calculating the distance of at least one portion of the chest (O₁) of the user (U) from said detecting device (123);
- and processing (102), by means of said logic control unit (C), at least one second group of said plurality of distance values of at least one portion of the body of said user (U), for calculating the distance of at least one portion of the right leg (O₂) of the user (U) from said detecting device (123);
- and processing (103), by means of said logic control unit (C), at least one third group of said plurality of distance values of at least one portion of the body of said user (U), for calculating the distance of at least one portion of the left leg (O₃) of the user (U) from said detecting device (123);
**characterized**
**in that** said detecting device (123) is a distance sensor, configured to detect values and to output a packet of values comprising the distance values (O) of the chest, the right leg, the left leg of the user (U) during the training,
wherein said distance sensor (123) outputs said packet of values in the form of a matrix (M),
wherein said first group of said plurality of distance values of at least one portion of the body of said user (U) is contained in an upper portion (M1) of said matrix (M), said second group of said plurality of distance values of at least one portion of the body of said user (U) is contained in a first lower portion (M21) of said matrix (M), and said third group of said plurality of distance values of at least one portion of the body of said user (U) is contained in the cells of a second lower portion (M22) of said matrix (M), and
wherein the upper portion (M1) of the matrix (M) contains said first group of detected distance values to determine the distance of the chest (O₁), the first lower portion (M21) contains said second group of detected distance values to determine the distance of the right leg (O₂) of the user (U), and the second lower portion (M22) contains said third group of detected distance values to determine the distance of the left leg (O₃).

2. Method according to the preceding claim, **characterized in that** said processing step (101) comprises the following sub-steps:
- selecting (1011), by means of said logic control unit (C), said first group of said plurality of distance values of at least one portion of the body;
- calculating (1012), by means of said logic control unit (C), said distance of at least one portion of the chest (O₁) of the user (U) from said detecting device (123) as statistical data of said first group of values of said plurality of distance values of at least one portion of the body.

3. Method according to the preceding claim, **characterized in that** it comprises the following step:
- comparing (104), by means of said logic control unit (C), said current distance data of at least one portion of the chest (O1), calculated in said sub-step of calculating (1012), with a predetermined reference value (O_{1R}), corresponding to an optimal distance of the user (U) from said detecting device (123), on said exercise surface (111).

4. Method according to the preceding claim, **characterized in that** said step of comparing (104) comprises the following sub-steps:
- if said current distance data of at least one portion of the chest (O₁) is higher than said predetermined reference value (O_{1R}) plus a predetermined threshold value (S), then commanding the decrease (1041), by means of said logic control unit (C), of the advancement speed of said exercise surface (111),
- if said current distance data of at least one portion of the chest (O₁) is included in a range having as lower bound said predetermined reference value (O_{1R}) minus said predetermined threshold value (S) and as upper bound said predetermined reference value (O_{1R}) plus said predetermined threshold value (S), then leaving unchanged (1042) the advancement speed of said exercise surface (111);
- if said current distance data of at least one portion of the chest (O₁) is lower than said predetermined reference value (O_{1R}) minus a predetermined threshold value (S), then commanding the increase (1043), by means of said logic control unit (C), of the advancement speed of said exercise surface (111).

5. Method according to any one of the preceding claims, **characterized in that** said processing step (102) comprises the following sub-steps:
- selecting (1021), by means of said logic control unit (C), said second group of values of said plurality of distance values of at least one portion of the body;
- calculating (1022), by means of said logic control unit (C), said distance of at least one portion of the right leg (O₂) of the user (U) from said detecting device (123) as statistical data of said second group of values of said plurality of distance values of at least one portion of the body.

6. Method according to any one of the preceding claims, **characterized in that** said processing step (103) comprises the following sub-steps:
- selecting (1031), by means of said logic control unit (C), said third group of values of said plurality of distance values of at least one portion of the body;
- calculating (1032), by means of said logic control unit (C), said distance of at least one portion of the left leg (O₃) of the user (U) from said detecting device (123) as statistical data of said third group of values of said plurality of distance values of at least one portion of the body.

7. Method according to any one of claims 5 or 6, **characterized in that** it comprises the following step:
- providing (105), by means of a display (D), parameters of the execution of the gymnastic exercise, based on the distance data calculated in said sub-steps of calculating (1022; 1032).

8. Method according to the preceding claim, **characterized in that** said step of providing (105) parameters comprises the following sub-steps:
- obtaining the oscillation period (T_{D}) of the right leg, from said distance data of at least one portion of the right leg (O₂);
- obtaining the oscillation period (T_{S}) of the left leg, from said distance data of at least one portion of the left leg (O₃);
- comparing said oscillation period (T_{D}) of the right leg and said oscillation period (T_{S}) of the left leg;
- displaying the result of said comparison by means of a display (D) of said gymnastic machine (1).

9. Method according to any one of claims 7 or 8, **characterized in that** said step of providing (105) parameters comprises the following sub-steps:
- obtaining the amplitude of the oscillation (A_{S}) of the left leg, from said distance data of at least one portion of the left leg (O₃);
- obtaining the amplitude of the oscillation (A_{D}) of the right leg, from said distance data of at least one portion of the right leg (O₂);
- comparing said amplitude of the oscillation (A_{D}) of the right leg and said amplitude of the oscillation (A_{S}) of the left leg;
- displaying said amplitude of the oscillation (A_{D}) of the right leg and said amplitude of the oscillation (A_{S}) of the left leg or the result of said comparison by means of a display (D) of said gymnastic machine (1).

10. Method according to any one of claims 7-9, **characterized in that** said step of providing (105) parameters comprises the following sub-steps:
- obtaining the oscillation period (T_{D}) of the right leg, from said distance data of at least one portion of the right leg (O₂);
- obtaining the oscillation period (T_{S}) of the left leg, from said distance data of at least one portion of the left leg (O₃);
- calculating the running cadence (C) as the inverse of the time (T_{SD}) that passes between two successive peaks of the oscillation (T_{D}) of the right leg and of the oscillation (T_{S}) of the left leg;
- displaying said cadence (C) by means of a display (D) of said gymnastic machine (1).

11. Method according to any one of the preceding claims, **characterized in that** it comprises the following steps:
- detecting (200) a plurality of ambient brightness values;
- processing (201) said plurality of detected ambient brightness values for calculating an ambient brightness index (I_{L});
- comparing (202), said ambient brightness index (I_{L}), with a predetermined threshold value;
- if said ambient brightness index (I_{L}) is greater than said predetermined threshold value, carrying out the steps of the method according to any one of claims 1-10;
- if said ambient brightness index (I_{L}) is lower than said predetermined threshold value, activating a safety operating state for said gymnastic machine (1).

12. A computer program comprising instructions which, when executed by the logic control unit (C) of a gymnastic machine (1), cause the logic control unit (C) to receive the distance values detected by a detecting device (123) and to carry out the steps of the method according to any one of claims 1 to 11 which are performed by the logic control unit (C).

13. A computer-readable medium comprising the computer program according to claim 12.

14. A gymnastic machine (1) comprising:
- an exercise surface (111) for the training of a user (U) on said gymnastic machine (1), the exercise surface (111) having a development direction (L) and an advancement direction (V) parallel to the development direction (L) of the exercise surface (111);
- a logic control unit (C) of the movement of the exercise surface (111);
- a detecting device (123), operatively connected to said logic control unit (C), configured to detect at least one distance value (O) of a portion of the user (U) from said detecting device (123) itself, during the execution of the gymnastic exercise by the user (U) on the exercise surface (111);
- said logic control unit (C) being configured to carry out the method for detecting the distance of portions of the body of a user (U) from a part of a gymnastic machine (1) according to any one of claims 1-11.

## Patentansprüche

1. Verfahren zur Erfassung des Abstands von Körperteilen eines Benutzers (U) von
ein vorderer Teil (121) eines Turngeräts (1), der mit einer Erfassungsvorrichtung (123) versehen ist, die an dem vorderen Teil (121) angeordnet ist, sowie eine Logiksteuereinheit (C), die funktionsmäßig verbunden ist
an die genannte Erfassungsvorrichtung (123) während der Ausführung einer gymnastischen Übung auf einer Trainingsfläche (111), die so ausgelegt ist, dass sie sich entlang einer Vorwärtsrichtung mit einer Vorwärtsgeschwindigkeit bewegt, wobei das Verfahren die folgenden Schritte umfasst:
- Erfassen (100) mittels der Erfassungsvorrichtung (123) einer Vielzahl von Abstandswerten mindestens eines Körperteils des Benutzers (U) vom vorderen Teil (121) des Fitnessgeräts (1), wobei die Vielzahl von Abstandswerten mindestens eines Körperteils des Benutzers (U) in Form einer Matrix (M) festgestellt ist, in
wobei jeder Zelle mindestens ein Abstandswert (O) zugeordnet ist;
- Verarbeitung (101) mindestens einer ersten Gruppe der mehreren Abstandswerte mindestens eines Körperteils des Benutzers (U) mittels der genannten Logiksteuereinheit (C), um den Abstand mindestens eines Brustbereichs (O₁) des Benutzers (U) von der genannten Erfassungsvorrichtung (123) zu berechnen;
- und Verarbeiten (102) mittels der genannten Logiksteuereinheit (C) mindestens einer zweiten Gruppe der genannten Vielzahl von Abstandswerten mindestens eines Körperteils des genannten Benutzers (U), um den Abstand mindestens eines Teils des rechten Beins (O₂) des Benutzers (U) von der genannten Erfassungsvorrichtung (123) zu berechnen;
- und Verarbeiten (103) mittels der genannten Logiksteuereinheit (C) mindestens einer dritten Gruppe der genannten Vielzahl von Abstandswerten mindestens eines Körperteils des genannten Benutzers (U), um den Abstand mindestens eines Teils des linken Beins (O₃) des Benutzers (U) von der genannten Erfassungsvorrichtung (123) zu berechnen;
**dadurch gekennzeichnet**,
wobei die genannte Erfassungsvorrichtung (123) ein Abstandssensor ist, der so ausgelegt ist, dass er Werte erfasst und ein Wertepaket ausgibt, das die Abstandswerte (O) von Brust, rechtem Bein und linkem Bein des Benutzers (U) während des Trainings enthält, wobei der Abstandssensor (123) das Wertepaket in Form von eine Matrix (M),
wobei die erste Gruppe der mehreren Abstandswerte von mindestens einem ein Körperabschnitt des Benutzers (U) ist in einem oberen Abschnitt (M1) der Matrix (M) enthalten, wobei die zweite Gruppe der mehreren Abstandswerte mindestens eines Körperabschnitts des Benutzers (U) in einem ersten unteren Abschnitt (M21) der Matrix (M) enthalten ist, und die dritte Gruppe der Vielzahl von Abstandswerten mindestens eines Körperabschnitts des Benutzers (U) in den Zellen eines zweiten unteren Abschnitts (M22) der Matrix (M) enthalten ist, und
wobei der obere Abschnitt (M1) der Matrix (M) die erste Gruppe der erfassten Abstandswerte zur Bestimmung des Abstands der Brust (O₁) enthält, der erste untere Abschnitt (M21) die zweite Gruppe der erfassten Abstandswerte zur Bestimmung des Abstands des rechten Beins (O₂) des Benutzers (U) enthält und der Der zweite untere Abschnitt (M22) enthält die genannte dritte Gruppe erfasster Abstandswerte, um Bestimme die Länge des linken Beins (O₃).

2. Verfahren nach dem vorstehenden Anspruch, **dadurch gekennzeichnet, dass** der Verarbeitungsschritt (101) die folgenden Teilschritte umfasst:
- Auswählen (1011) der ersten Gruppe der mehreren Abstandswerte mindestens eines Körperabschnitts mittels der genannten Logiksteuereinheit (C);
- Berechnung (1012) des Abstands mindestens eines Brustbereichs (O₁) des Benutzers (U) von der Erfassungsvorrichtung (123) mittels der genannten Logiksteuereinheit (C) als statistische Daten der genannten ersten Gruppe von Werten aus der genannten Vielzahl von Abstandswerten mindestens eines Körperbereichs.

3. Verfahren nach dem vorstehenden Anspruch, **dadurch gekennzeichnet, dass** es den folgenden Schritt umfasst:
- Vergleichen (104) der im Teilschritt der Berechnung (1012) ermittelten aktuellen Abstandsdaten mindestens eines Brustbereichs (01) mittels der Logiksteuereinheit (C) mit einem vorgegebenen Referenzwert (O_{1R}), der einem optimalen Abstand des Benutzers (U) von der Erfassungsvorrichtung (123) entspricht, auf der Trainingsfläche (111).

4. Verfahren nach dem vorstehenden Anspruch, **dadurch gekennzeichnet, dass** der Vergleichsschritt (104) die folgenden Teilschritte umfasst:
- wenn die genannten aktuellen Abstandsdaten mindestens eines Bereichs des Brustkorbs (O₁) höher sind als der genannte vorbestimmte Referenzwert (O_{1R}) zuzüglich eines vorbestimmten Schwellenwerts (S), dann Anweisen der Verringerung (1041) mittels der genannten Logiksteuereinheit (C), der Vorschubgeschwindigkeit der genannten Trainingsfläche (111),
- wenn die genannten aktuellen Abstandsdaten mindestens eines Bereichs der Brust (O₁) in einem Bereich liegen, dessen untere Grenze aus dem genannten vorgegebenen Referenzwert (O_{1R}) abzüglich des genannten vorgegebenen Schwellenwerts (S) und dessen obere Grenze aus dem genannten vorgegebenen Referenzwert (O_{1R}) zuzüglich des genannten vorgegebenen Schwellenwerts (S) besteht, dann wird Unverändert (1042) die Vorschubgeschwindigkeit der genannten Trainingsfläche (111);
- wenn die genannten aktuellen Abstandsdaten mindestens eines Bereichs des Brustkorbs (O₁) kleiner sind als der genannte vorgegebene Referenzwert (O_{1R}) abzüglich eines vorgegebenen Schwellenwerts (S), dann wird der Befehl erteilt die Erhöhung (1043) der Vorschubgeschwindigkeit der Trainingsfläche (111) mittels der genannten Logiksteuereinheit (C).

5. Verfahren nach einem der vorstehenden Ansprüche, **dadurch gekennzeichnet, dass** der Verarbeitungsschritt (102) die folgenden Teilschritte umfasst:
- Auswählen (1021) der zweiten Gruppe von Werten aus der Vielzahl von Abstandswerten mindestens eines Körperabschnitts mittels der genannten Logiksteuereinheit (C);
- Berechnung (1022) des Abstands mindestens eines Abschnitts des rechten Beins (O₂) des Benutzers (U) von der Erfassungsvorrichtung (123) mittels der genannten Logiksteuereinheit (C) als statistische Daten der genannten zweiten Gruppe von Werten aus der genannten Vielzahl von Abstandswerten mindestens eines Körperabschnitts.

6. Verfahren nach einem der vorstehenden Ansprüche, **dadurch gekennzeichnet, dass** der Verarbeitungsschritt (103) die folgenden Teilschritte umfasst:
- Auswählen (1031) der dritten Gruppe von Werten aus der Vielzahl von Abstandswerten mindestens eines Körperabschnitts mittels der genannten Logiksteuereinheit (C);
- Berechnung (1032) des Abstands mindestens eines Abschnitts des linken Beins (O₃) des Benutzers (U) von der Erfassungsvorrichtung (123) mittels der genannten Logiksteuereinheit (C) als statistische Daten der genannten dritten Gruppe von Werten aus der genannten Vielzahl von Abstandswerten mindestens eines Körperabschnitts.

7. Verfahren nach einem der Ansprüche 5 oder 6, **dadurch gekennzeichnet, dass** es den folgenden Schritt umfasst:
- Bereitstellung (105) von Parametern zur Durchführung der Gymnastikübungen über ein Display (D)
Vorgang, basierend auf den in den genannten Teilschritten der Berechnung (1022; 1032) ermittelten Entfernungsdaten.

8. Verfahren nach dem vorstehenden Anspruch, **dadurch gekennzeichnet, dass** der Schritt des Bereitstellens (105) von Parametern
umfasst die folgenden Teilschritte:
- Ermittlung der Schwingungsperiode (T_{D}) des rechten Beins anhand der genannten Abstandsdaten mindestens eines Abschnitts des rechten Beins (O₂);
- Ermittlung der Schwingungsperiode (Tₛ) des linken Beins anhand der genannten Abstandsdaten mindestens eines Abschnitts des linken Beins (O₃);
- Vergleich der Schwingungsperiode (T_{D}) des rechten Beins mit der Schwingungsperiode (Tₛ) des linken Beins;
- Anzeige des Ergebnisses dieses Vergleichs über eine Anzeige (D) des Turngeräts (1).

9. Verfahren nach einem der Ansprüche 7 oder 8, **dadurch gekennzeichnet, dass** der Schritt des Bereitstellens (105) von Parametern
umfasst die folgenden Teilschritte:
- Ermittlung der Schwingungsamplitude (Aₛ) des linken Beins anhand der genannten Abstandsdaten mindestens eines Abschnitts des linken Beins (O₃);
- Ermittlung der Schwingungsamplitude (A_{D}) des rechten Beins anhand der genannten Abstandsdaten mindestens eines Abschnitts des rechten Beins (O₂);
- Vergleich der Schwingungsamplitude (A_{D}) des rechten Beins mit der Schwingungsamplitude (Aₛ) des linken Beins;
- Anzeige der Schwingungsamplitude (A_{D}) des rechten Beins und der Schwingungsamplitude (Aₛ) des linken Beins oder des Ergebnisses dieses Vergleichs mittels einer Anzeige (D) des Fitnessgeräts (1).

10. Verfahren nach einem der Ansprüche 7-9, **dadurch gekennzeichnet, dass** der Schritt des Bereitstellens (105) von Parametern
umfasst die folgenden Teilschritte:
- Ermittlung der Schwingungsperiode (T_{D}) des rechten Beins anhand der genannten Abstandsdaten mindestens eines Abschnitts des rechten Beins (O₂);
- Ermittlung der Schwingungsperiode (Tₛ) des linken Beins anhand der genannten Abstandsdaten mindestens eines Abschnitts des linken Beins (O₃);
- Berechnung der Laufkadenz (C) als Kehrwert der Zeit (T_{SD}), die zwischen zwei aufeinanderfolgenden Spitzenwerten der Schwingung (T_{D}) des rechten Beins und der Schwingung (Tₛ) des linken Beins vergeht;
- Anzeige der genannten Kadenz (C) über eine Anzeige (D) des genannten Fitnessgeräts (1).

11. Verfahren nach einem der vorstehenden Ansprüche, **dadurch gekennzeichnet, dass** es die folgenden Schritte umfasst:
- Erfassen (200) einer Vielzahl von Umgebungshelligkeitswerten;
Verarbeitung (201) der mehreren erfassten Umgebungshelligkeitswerte zur Berechnung eines Umgebungshelligkeitsindex (I_{L});
Vergleichen (202) desgenannten Umgebungshelligkeitsindex (I_{L}) mit einem vorgegebenen Schwellenwert;
wenn der Umgebungshelligkeitsindex (I_{L}) größer ist als der vorbestimmte Schwellenwert, werden die Schritte des Verfahrens gemäß einem der Ansprüche 1-10 durchgeführt;
- wenn der genannte Umgebungshelligkeitsindex (I_{L}) unter dem genannten vorgegebenen Schwellenwert liegt, wird ein Sicherheitsbetriebszustand für das genannte Turngerät (1) aktiviert.

12. Computerprogramm, das Befehle umfasst, die, wenn sie von der Logiksteuereinheit (C) eines Fitnessgeräts (1) ausgeführt werden, bewirken, dass die Logiksteuereinheit (C) die von einer Erfassungsvorrichtung (123) erfassten Abstandswerte empfängt und die Schritte des Verfahrens gemäß einem der Ansprüche 1 bis 11 ausführt, die von der Logiksteuereinheit (C) durchgeführt werden.

13. computerlesbares Medium, das das Computerprogramm gemäß Anspruch 12 enthält.

14. Turngerät (1), umfassend:
- eine Trainingsfläche (111) für das Training eines Benutzers (U) auf dem genannten Fitnessgerät (1), wobei die Trainingsfläche (111) eine Ausdehnungsrichtung (L) und eine Vorwärtsbewegung (V) parallel zur Ausdehnungsrichtung (L) der Trainingsfläche (111) aufweist;
- eine Logiksteuereinheit (C) für die Bewegung der Trainingsfläche (111);
- eine Erfassungsvorrichtung (123), die funktionsmäßig mit der Logiksteuereinheit (C) verbunden ist,
so konfiguriert, dass während der Ausführung der Gymnastikübung durch den Benutzer (U) auf der Trainingsfläche (111) mindestens ein Abstandswert (O) eines Körperteils des Benutzers (U) von der Erfassungsvorrichtung (123) selbst erfasst wird;
- wobei die genannte Logiksteuereinheit (C) so konfiguriert ist, dass sie das Verfahren zur Erfassen des Abstands von Körperteilen eines Benutzers (U) zu einem Teil eines Turngeräts (1) gemäß einem der Ansprüche 1-11.

## Revendications

1. Procédé de détection de la distance de parties du corps d'un utilisateur (U) et
une partie avant (121) d'un appareil de gymnastique (1) équipée d'un dispositif de détection (123), disposé sur ladite partie avant (121), et d'une unité de commande logique (C), reliée de manière fonctionnelle
vers ledit dispositif de détection (123), pendant l'exécution d'un exercice de gymnastique sur une surface d'exercice (111) configurée pour se déplacer selon une direction d'avancement à une vitesse d'avancement, ledit procédé comprenant les étapes suivantes:
- détecter (100), à l'aide dudit dispositif de détection (123), une pluralité de valeurs de distance entre au moins une partie du corps dudit utilisateur (U) et ladite partie avant (121) de ladite machine de gymnastique (1), ladite pluralité de valeurs de distance entre au moins une partie du corps dudit utilisateur (U) étant détecté sous la forme d'une matrice (M), dans
à chaque cellule étant associée au moins une valeur de distance (O);
- le traitement (101), au moyen de ladite unité de commande logique (C), d'au moins un premier groupe parmi ladite pluralité de valeurs de distance d'au moins une partie du corps dudit utilisateur (U), afin de calculer la distance entre au moins une partie de la poitrine (O₁) de l'utilisateur (U) et ledit dispositif de détection (123);
- et le traitement (102), au moyen de ladite unité de commande logique (C), d'au moins un deuxième groupe parmi ladite pluralité de valeurs de distance d'au moins une partie du corps dudit utilisateur (U), afin de calculer la distance entre au moins une partie de la jambe droite (O₂) de l'utilisateur (U) et ledit dispositif de détection (123);
- et le traitement (103), au moyen de ladite unité de commande logique (C), d'au moins un troisième groupe parmi ladite pluralité de valeurs de distance d'au moins une partie du corps dudit utilisateur (U), afin de calculer la distance entre au moins une partie de la jambe gauche (O₃) de l'utilisateur (U) et ledit dispositif de détection (123);
**caractérisé en ce que**
dans la mesure où ledit dispositif de détection (123) est un capteur de distance, configuré pour détecter des valeurs et pour transmettre un paquet de valeurs comprenant les valeurs de distance (O) de la poitrine, de la jambe droite et de la jambe gauche de l'utilisateur (U) pendant l'entraînement,
dans lequel ledit capteur de distance (123) transmet ledit ensemble de valeurs sous la forme d'une matrice (M),
dans lequel ledit premier groupe de ladite pluralité de valeurs de distance d'au moins un une partie du corps dudit utilisateur (U) est contenue dans une partie supérieure (M1) de ladite matrice (M), ledit deuxième groupe de ladite pluralité de valeurs de distance d'au moins une partie du corps dudit utilisateur (U) est contenu dans une première partie inférieure (M21) de ladite matrice (M), et ledit troisième groupe de ladite pluralité de valeurs de distance d'au moins une partie du corps dudit utilisateur (U) est contenu dans les cellules d'une deuxième partie inférieure (M22) de ladite matrice (M), et
dans lequel la partie supérieure (M1) de la matrice (M) contient ledit premier groupe de valeurs de distance détectées permettant de déterminer la distance de la poitrine (O₁), la première partie inférieure (M21) contient ledit deuxième groupe de valeurs de distance détectées permettant de déterminer la distance de la jambe droite (O₂) de l'utilisateur (U), et la deuxième partie inférieure (M22) contient ledit troisième groupe de valeurs de distance détectées pour déterminer la longueur de la jambe gauche (O₃).

2. Procédé selon la revendication précédente, **caractérisé en ce que** ladite étape de traitement (101) comprend les sous-étapes suivantes:
- sélectionner (1011), à l'aide de ladite unité de commande logique (C), ledit premier groupe parmi ladite pluralité de valeurs de distance d'au moins une partie du corps;
- calculer (1012), à l'aide de ladite unité de commande logique (C), ladite distance entre au moins une partie du thorax (O₁) de l'utilisateur (U) et ledit dispositif de détection (123), en tant que données statistiques dudit premier groupe de valeurs parmi ladite pluralité de valeurs de distance d'au moins une partie du corps.

3. Procédé selon la revendication précédente, **caractérisé en ce qu'**il comprend l'étape suivante:
- comparer (104), au moyen de ladite unité de commande logique (C), lesdites données de distance actuelle d'au moins une partie du thorax (O1), calculées lors de ladite sous-étape de calcul (1012), à une valeur de référence prédéterminée (O_{1R}), correspondant à une distance optimale entre l'utilisateur (U) et ledit dispositif de détection (123), sur ladite surface d'exercice (111).

4. Procédé selon la revendication précédente, **caractérisé en ce que** ladite étape de comparaison (104) comprend les sous-étapes suivantes:
- si lesdites données de distance actuelles d'au moins une partie de la poitrine (O₁) sont supérieures à ladite valeur de référence prédéterminée (O_{1R}) majorée d'une valeur seuil prédéterminée (S), alors ordonner la diminution (1041), au moyen de ladite unité de commande logique (C), de la vitesse d'avance de ladite surface d'exercice (111),
- si lesdites données de distance actuelles d'au moins une partie de la poitrine (O₁) se situent dans un intervalle dont la borne inférieure est égale à ladite valeur de référence prédéterminée (O_{1R}) moins ladite valeur seuil prédéterminée (S) et dont la borne supérieure est égale à ladite valeur de référence prédéterminée (O_{1R}) plus ladite valeur seuil prédéterminée (S), alors inchangée (1042), la vitesse d'avance de ladite surface d'exercice (111);
- si lesdites données de distance actuelles d'au moins une partie du thorax (O₁) sont inférieures à ladite valeur de référence prédéterminée (O_{1R}) moins une valeur seuil prédéterminée (S), alors ordonner l'augmentation (1043), par l'intermédiaire de ladite unité de commande logique (C), de la vitesse d'avance de ladite surface d'exercice (111).

5. Procédé selon l'une quelconque des revendications précédentes, **caractérisé en ce que** ladite étape de traitement (102) comprend les sous-étapes suivantes:
- sélectionner (1021), à l'aide de ladite unité de commande logique (C), ledit deuxième groupe de valeurs parmi ladite pluralité de valeurs de distance d'au moins une partie du corps;
- calculer (1022), à l'aide de ladite unité de commande logique (C), ladite distance entre au moins une partie de la jambe droite (O₂) de l'utilisateur (U) et ledit dispositif de détection (123), en tant que données statistiques dudit deuxième groupe de valeurs parmi ladite pluralité de valeurs de distance d'au moins une partie du corps.

6. Procédé selon l'une quelconque des revendications précédentes, **caractérisé en ce que** ladite étape de traitement (103) comprend les sous-étapes suivantes:
- sélectionner (1031), à l'aide de ladite unité de commande logique (C), ledit troisième groupe de valeurs parmi ladite pluralité de valeurs de distance d'au moins une partie du corps;
- calculer (1032), à l'aide de ladite unité de commande logique (C), ladite distance entre au moins une partie de la jambe gauche (O₃) de l'utilisateur (U) et ledit dispositif de détection (123), en tant que données statistiques dudit troisième groupe de valeurs parmi ladite pluralité de valeurs de distance d'au moins une partie du corps.

7. Procédé selon l'une quelconque des revendications 5 ou 6, **caractérisé en ce qu'**il comprend l'étape suivante:
- afficher (105), au moyen d'un écran (D), les paramètres relatifs à l'exécution des exercices de gymnastique
opération, sur la base des données de distance calculées au cours desdites sous-étapes de calcul (1022; 1032).

8. Procédé selon la revendication précédente, **caractérisé en ce que** ladite étape consistant à fournir (105) des paramètres
comprend les étapes suivantes:
- déterminer la période d'oscillation (T_{D}) de la jambe droite, à partir desdites données de distance relatives à au moins une partie de la jambe droite (O₂);
- déterminer la période d'oscillation (Tₛ) de la jambe gauche, à partir desdites données de distance relatives à au moins une partie de la jambe gauche (O₃);
- comparer ladite période d'oscillation (T_{D}) de la jambe droite et ladite période d'oscillation (Tₛ) de la jambe gauche;
- afficher le résultat de ladite comparaison au moyen d'un écran (D) de ladite machine de gymnastique (1).

9. Procédé selon l'une quelconque des revendications 7 ou 8, **caractérisé en ce que** ladite étape de fourniture (105) de paramètres
comprend les étapes suivantes:
- déterminer l'amplitude de l'oscillation (Aₛ) de la jambe gauche, à partir desdites données de distance d'au moins une partie de la jambe gauche (O₃);
- déterminer l'amplitude de l'oscillation (A_{D}) de la jambe droite, à partir desdites données de distance relatives à au moins une partie de la jambe droite (O₂);
- comparer ladite amplitude d'oscillation (A_{D}) de la jambe droite et ladite amplitude d'oscillation (Aₛ) de la jambe gauche;
- afficher ladite amplitude d'oscillation (A_{D}) de la jambe droite et ladite amplitude d'oscillation (Aₛ) de la jambe gauche, ou le résultat de ladite comparaison, au moyen d'un écran (D) de ladite machine de gymnastique (1).

10. Procédé selon l'une quelconque des revendications 7-9, **caractérisé en ce que** ladite étape de fourniture (105) de paramètres
comprend les étapes suivantes:
- déterminer la période d'oscillation (T_{D}) de la jambe droite, à partir desdites données de distance relatives à au moins une partie de la jambe droite (O₂);
- déterminer la période d'oscillation (Tₛ) de la jambe gauche, à partir desdites données de distance relatives à au moins une partie de la jambe gauche (O₃);
- calculer la cadence de course (C) comme l'inverse du temps (T_{SD}) qui s'écoule entre deux pics successifs de l'oscillation (T_{D}) de la jambe droite et de l'oscillation (Tₛ) de la jambe gauche;
- l'affichage de ladite cadence (C) au moyen d'un écran (D) de ladite machine de gymnastique (1).

11. Procédé selon l'une quelconque des revendications précédentes, **caractérisé en ce qu'**il comprend les étapes suivantes:
- détecter (200) une pluralité de valeurs de luminosité ambiante;
traitement (201) de ladite pluralité de valeurs de luminosité ambiante détectées afin de calculer un indice de luminosité ambiante (I_{L});
en comparant (202) ledit indice de luminosité ambiante (I_{L}) à une valeur seuil prédéterminée;
si ledit indice de luminosité ambiante (I_{L}) est supérieur à ladite valeur seuil prédéterminée, mettre en œuvre les étapes du procédé selon l'une quelconque des revendications 1-10;
- si ledit indice de luminosité ambiante (I_{L}) est inférieur à ladite valeur seuil prédéterminée, activer un mode de fonctionnement de sécurité pour ledit appareil de gymnastique (1).

12. Programme informatique comprenant des instructions qui, lorsqu'elles sont exécutées par l'unité de commande logique (C) d'un appareil de gymnastique (1), amènent ladite unité de commande logique (C) à recevoir les valeurs de distance détectées par un dispositif de détection (123) et à mettre en œuvre les étapes du procédé selon l'une quelconque des revendications 1 à 11, lesquelles sont exécutées par l'unité de commande logique (C).

13. Support lisible par ordinateur comprenant le programme informatique selon la revendication 12.

14. Appareil de gymnastique (1) comprenant:
- une surface d'exercice (111) destinée à l'entraînement d'un utilisateur (U) sur ladite machine de gymnastique (1), la surface d'exercice (111) présentant une direction de développement (L) et une direction d'avancement (V) parallèle à la direction de développement (L) de la surface d'exercice (111);
- une unité de commande logique (C) du déplacement de la surface d'exercice (111);
- un dispositif de détection (123), relié de manière fonctionnelle à ladite unité de commande logique (C), conçu pour détecter au moins une valeur de distance (O) entre une partie du corps de l'utilisateur (U) et ledit dispositif de détection (123) lui-même, pendant l'exécution de l'exercice de gymnastique par l'utilisateur (U) sur la surface d'exercice (111)a;
- ladite unité de commande logique (C) étant configurée pour mettre en œuvre le procédé visant à détecter la distance entre certaines parties du corps d'un utilisateur (U) et une partie d'un appareil de gymnastique (1), selon l'une quelconque des revendications 1-11.
